# EUROPEAN PATENT APPLICATION

(11) **EP 4 115 901 A1**
(43) Date of publication of application: **11.01.2023**
(21) Application number: 21765344.3
(22) Date of filing: 26.02.2021
(51) Int. Cl.: A61K 39/39, A61K 9/19, A61K 39/00

(54) **LIVE-PATHOGEN-MIMETIC NANOPARTICLES BASED ON PATHOGEN CELL WALL SKELETON, AND PRODUCTION METHOD THEREOF**

(30) Priority: 02.03.2020 KR 20200025780; 25.02.2021 KR 20210026013
(71) Applicant: PROGENEER INC., Guro-gu Seoul 08380 (KR)
(72) Inventor: LIM, Yong Taik, Seongnam-si, Gyeonggi-do 13597 (KR); LEE, Sang Nam, Suwon-si, Gyeonggi-do 16417 (KR)
(74) Representative: Dehns
(86) International application number: PCT/KR2021/002508
(87) International publication number: WO 2021/177679

(57) **Abstract**

The present invention relates to: a lyophilized preparation of a pathogen cell wall skeleton, the preparation containing the pathogen cell wall skeleton as an active ingredient; various live-pathogen-mimetic nanoparticles produced by using the lyophilized preparation and antagonists of toll-like receptor 7 or 8 which can induce the efficacy of the live pathogen; a use thereof; and a production method thereof.

## Description

### [Technical Field]

The present invention relates to a live pathogen-mimetic nano-molecule based on a pathogen cell wall skeleton and a method of manufacturing the same, and particularly, to a live pathogen-mimetic nano-molecule based on a pathogen cell wall skeleton, manufactured using a freeze-dried formulation of a pathogen cell wall skeleton easily dispersed in an aqueous solution and an immune activation material that can induce live pathogen efficacy, a use thereof, and a method of manufacturing the same.

This application claims priority to and the benefit of Korean Patent Application No. 10-2020-0025780 filed in the Korean Intellectual Property Office on March 2, 2020 and Korean Patent Application No. 10-2021-0026013 filed in the Korean Intellectual Property Office on February 25, 2021, and all the contents disclosed in the specification and drawings of that application are incorporated in this application.

### [Background Art]

A vaccine is a drug that imparts acquired immunity to animals, including humans, and is widely used to prevent or treat microbial infectious diseases as well as malignant tumors (cancer), allergic diseases, and the like using substances capable of inducing immunogenicity such as dead or attenuated pathogens, proteins, synthetic peptides, or the like. In general, there are live vaccines, live attenuated vaccines, dead vaccines, and recombinant vaccines, and among these, live vaccines and live attenuated vaccines not only take a long time to produce viruses and have a high production cost, but also have safety issues. Accordingly, studies on recombinant vaccines with excellent safety and high production efficiency have been actively conducted, but in the case of recombinant vaccines, compared with existing virus-derived vaccines, immunogenicity is low and thus treatment efficiency decreases (Korean Publication Patent No. 10-2014-0041134). In order to overcome these shortcomings, interest in the development of new alternative pharmaceuticals using the pathogen cell wall skeleton has recently increased, but most pharmaceutical preparations using the cell wall skeleton have the following problems. First, the cell wall skeletons of most pathogens show fat-soluble properties, so there is a limit to developing them into various pharmaceutical formulations. Second, vaccine components using the pathogen cell wall skeleton are superior in safety compared to killed or inactivated vaccines or live attenuated vaccines, but have a fatal limitation of weak immunogenicity. Therefore, to develop effective immunotherapeutic agents, it is urgently needed to develop a novel concept of pathogen-based vaccine, which can be mass-produced, has high stability and excellent immunogenicity, and can be produced in various formulations at the same time.

### [Disclosure]

### [Technical Problem]

The present invention has been devised to solve the aforementioned problems in the related art, and is directed to providing a live pathogen-mimetic nano-molecule, a use thereof, and a method of manufacturing the same, which can be mass-produced, has high stability and excellent immunogenicity, and can be produced in various formulations at the same time.

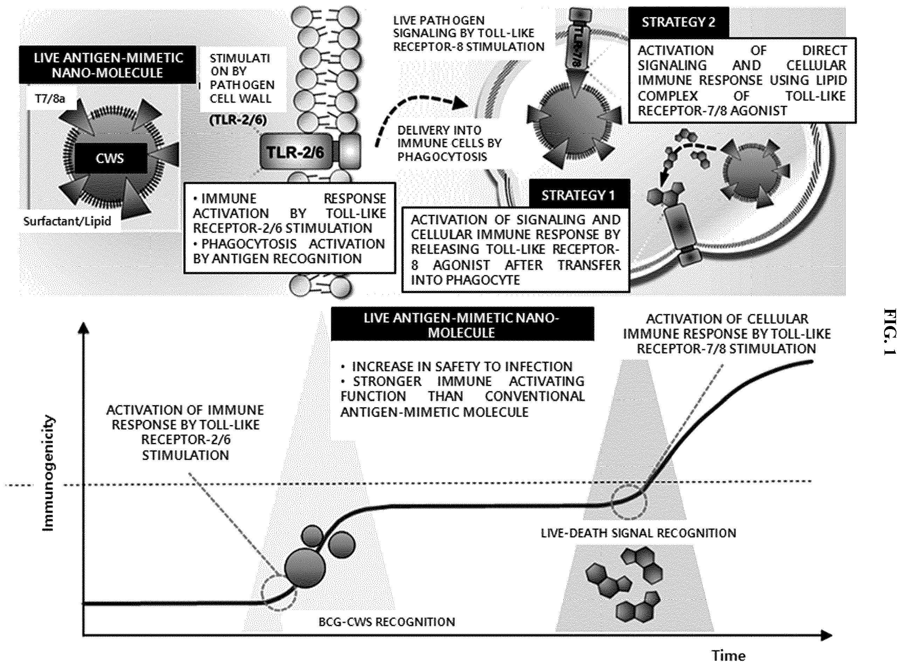

A live pathogen-mimetic nano-molecule including a pathogen cell wall skeleton, and a toll-like receptor 7 or 8 agonist first activates the immune response of cells by pathogen recognition based on the pathogen cell wall skeleton, and after being delivered into immune cells through phagocytosis, the toll-like receptor 7 or 8 agonist related to live/death signals of a live pathogen is operated to activate signaling and cellular immunity, thereby increasing immunogenicity, and therefore, a live pathogen-mimetic nano-molecule having high immunogenicity can be provided (FIG. 1).

However, technical problems to be solved in the present invention are not limited to the above-described problems, and other problems which are not described herein will be fully understood by those of ordinary skill in the art from the following descriptions.

### [Technical Solution]

The present invention provides a live pathogen-mimetic nano-molecule, which includes a toll-like receptor 7 or 8 agonist, and a pathogen cell wall skeleton-based nanodispersion. The toll-like receptor 7 or 8 agonist is preferably in an inactivated state by binding of a lipid to an activation site. The lipid preferably includes fatty acids, steroids, glycerides, phospholipids, cholesterols, and fat-soluble vitamins, and the lipid binds to the activation site of the toll-like receptor 7 or 8 agonist to make it inactive, and prevents the toll-like receptor 7 or 8 agonist from being absorbed into the body through a blood vessel, so there is no limitation as long as the type of lipid is known.

In addition, the pathogen cell wall skeleton-based nanodispersion refers to a form in which the pathogen cell outer wall component from which lipids and membrane proteins have been removed is dispersed in an aqueous solution, and the aqueous solution encompasses all types of solutions prepared using water as a solvent. The aqueous solution is preferably a buffer, a cationic buffer solution, or there is no limitation as long as water is used as a solvent.

In the present invention, the pathogen cell wall skeleton may vary according to species and a strain, and encompasses insoluble residues obtained through a purification process including nuclease, protease and organic solvent washing after physically disrupting pathogens. The pathogen cell wall skeleton generally consists of a biopolymer material, and may include, for example, a long-chain fatty acid, a sugar chain, and a peptidoglycan. However, there is no limitation as long as it is a factor that is generally included in the pathogen cell wall skeleton.

In another embodiment of the present invention, the pathogen cell wall skeleton of the present invention is lyophilized and easily dispersed in an aqueous solution.

In still another embodiment of the present invention, the pathogen encompasses microorganisms causing diseases, and preferably, pathogenic bacteria, viruses, and fungi, and more preferably, the Bacille-Calmette-Guerin (BCG) strain, bacteria of the genus Mycobacterium, bacteria of the genus Nocardia, bacteria of the genus Corynebacterium, bacteria of the genus Rhodococcus, bacteria of the genus Gordona, coronaviruses, but the present invention is not limited thereto.

In yet another embodiment of the present invention, the binding between the toll-like receptor 7 or 8 agonist and the lipid is a cleavable form, and the cleavable binding is preferably a carbamate, disulfide, ester, peptide, azide, amide, hydrazone, thioether, phosphodiester, or thioketal bond, or a combination thereof, and there is no limitation as long as it is a form in which a chemical bond at the binding site is cleaved in response to a tumor microenvironment, or the enzymes and pH of endosomes and lysosomes in cells, or a form in which a chemical bond at the binding site is cleaved by a specific stimulus such as a temperature, a redox potential, ROS, ATP, ultrasound, a magnetic field, or light. The function of the toll-like receptor 7 or 8 agonist is dynamically recovered within 4 days after the activation site is exposed by the cleavage. There is no limitation as long as the enzymes are any of various enzymes present in cells, and the enzyme is preferably an acid phosphatase, an acid pyrophosphate, a phosphodiesterase, a phosphoprotein phosphatase, a phosphatidic acid phosphatase, an arylsulfatase, a protease, a cathepsin, a collagenase, an arylamidase, a peptidase, an acid ribonuclease, an acid deoxyribonuclease, a lipase, a triglyceride lipase, a phospholipase, an esterase, a carboxyesterase, a clucocerebrosidase, a galactocerebrosidase, a sphingomyelinase, a glycosidase, an α-glucosidase, a β-glucosidase, a β-galactosidase, an α-mannosidase, an α-ucosidase, a β-xylosidase, an α-N-acetylhexosaminidase, a β-N-acetylhexosaminidase, a sialidase, a lysozyme, a hyaluronidase, or a β-glucuronidase.

In yet another embodiment of the present invention, the toll-like receptor 7 or 8 agonist is preferably an imidazoquinoline-, hydroxyadenine-, pteridine-, aminopyrimidine-, benzoazepine-, thia-oxoguanosine-based compound, or a derivative thereof, and there is no limitation as long as it is a known toll-like receptor 7 or 8 agonist.

In yet another embodiment of the present invention, the nano-molecule may be manufactured by forming a spherical nanoparticle using a pathogen cell wall skeleton-based nanodispersion, and binding the toll-like receptor 7 or 8 agonist to the spherical nanoparticle, enclosing it regardless of binding, attaching it to the surface of the nanoparticle, inserting it into the nanoparticle structure, or binding it by electrostatic interaction, and it is not limited as long as it is a form that can be included in the nano-molecule of the present invention.

In yet another embodiment of the present invention, the nano-molecule has a diameter of preferably 20 to 500 nm, and more preferably 50 to 300 nm.

In yet another embodiment of the present invention, the nano-molecule may be a nanoliposome, nanoemulsion, nanomicelle, or polymer nanoparticle form.

In yet another embodiment of the present invention, the nano-molecule may further include a material for activating humoral immunity, a material for activating cellular immunity, an immunostimulatory agent, an adjuvant in addition to the cell outer wall component, but the present invention is not limited thereto. Preferably, the nano-molecule further includes a toll-like receptor 7 or 8 agonist, a toll-like receptor agonist, a saponin, an antiviral peptide, an inflammasome inducer, a NOD ligand, a cytosolic DNA sensor (CDS) ligand, a stimulator of interferon genes (STING) ligand, an antigen, an emulsion, alum, a chemotherapeutic agent, an immune checkpoint inhibitor, or a combination thereof.

In addition, the present invention provides an adjuvant composition, which includes the nano-molecule as an active ingredient.

In addition, the present invention provides a vaccine composition, which includes the adjuvant composition and an antigen.

In one embodiment of the present invention, the antigen is preferably a protein, a recombinant protein, a glycoprotein, a gene, a peptide, a polysaccharide, a lipopolysaccharide, a polynucleotide, cells, a cell lysate, bacteria, a virus, or a cancer antigen, but there is no limitation as long as it is a commonly known antigen.

In another embodiment of the present invention, the vaccine composition may be used to prevent or treat a disease such as a microorganism-mediated infectious disease, tuberculosis or cancer, and the cancer prevention or treatment inhibits cancer proliferation, metastasis or recurrence, or inhibits resistance to anticancer therapy, and there is no limitation as long as it is a generally used cancer treatment method.

In still another embodiment of the present invention, the cancer may be bladder cancer, breast cancer, colorectal cancer, rectal cancer, lung cancer, colon cancer, thyroid cancer, oral cancer, pharyngeal cancer, laryngeal cancer, cervical cancer, brain cancer, ovarian cancer, kidney cancer, liver cancer, pancreatic cancer, prostate cancer, skin cancer, tongue cancer, uterine cancer, stomach cancer, bone cancer, or blood cancer, but the present invention is not limited thereto.

In yet another embodiment of the present invention, the vaccine composition may further include a chemotherapeutic agent or an immune checkpoint inhibitor.

In addition, the present invention provides a method of preventing and/or treating a disease, which includes administering a composition including the nano-molecule as an active ingredient into a subject.

In addition, the present invention provides a use of a composition including the nano-molecule as an active ingredient for preventing and/or treating a disease.

In addition, the present invention provides a use of the nano-molecule for producing a drug used in preventing and/or treating a disease.

In one embodiment of the present invention, the disease is preferably an infection disease, tuberculosis or cancer, but since the nano-molecule of the present invention is included, there is no limitation as long as it is a disease that is conventionally known to be prevented or treated using a vaccine, because the nano-molecule can increase immunogenicity effectively.

In addition, the present invention provides a method of manufacturing a live pathogen-mimetic nano-molecule, which includes: (a) disrupting pathogens; (b) removing a lipid from the resulting lysate; (c) removing a membrane protein from the lipid-removed lysate; (d) isolating a pathogen cell wall skeleton by centrifuging the membrane protein-removed lysate; (e) preparing a freeze-dried preparation by freeze-drying the isolated pathogen cell wall skeleton; (f) mixing the freeze-dried preparation, a surfactant and a positively-charged buffer solution; and (g) manufacturing a nano-molecule by adding a lipid, a lipid conjugate, or a toll-like receptor 7 or 8 agonist in which a lipid binds to an activation site to the mixture obtained in (f) and performing ultrasonication.

In one embodiment of the present invention, as a surfactant used to stably disperse the freeze-dried preparation of (f) in an aqueous solution, representatively, sodium dodecyl sulfate (SDS), didodecyldimethylammonium bromide (DMAB), Pluronic F68, Pluronic F127, polyvinyl alcohol (PVA), Tween-80, Span-85, oleic acid, and a combination thereof may be used, but there is no limitation as long as it is a generally known type of surfactant.

In another embodiment of the present invention, as a positively-charged buffer solution used to stably disperse the freeze-dried preparation of (f) in an aqueous solution, representatively, an L-lysine (L-Lys) buffer solution, an L-arginine (L-Arg) buffer solution, an L-histidine (L-His) buffer solution, an L-tyrosine (L-Tyr) buffer solution, an L-aspartic acid (L-Asp) buffer solution, an L-glutamine (L-Glu) buffer solution, or a combination thereof may be used, but there is no limitation as long as it is a generally known type of positively-charged buffer solution.

In still another embodiment of the present invention, the manufacturing method may further include, after (g), further adding any one or more selected from the group consisting of a toll-like receptor 7 or 8 agonist, a toll-like receptor agonist, a saponin, an antiviral peptide, an inflammasome inducer, a NOD ligand, a cytosolic DNA sensor (CDS) ligand, a stimulator of interferon genes (STING) ligand, an antigen, an emulsion, alum, a chemotherapeutic agent, an immune checkpoint inhibitor, and a combination thereof, performing mixing and sonication.

### [Advantageous Effects]

A freeze-dried preparation of a pathogen cell wall skeleton including a lipid- and membrane protein-removed pathogen cell wall skeleton according to the present invention as an active ingredient, that is, a nanodispersion based on a pathogen cell wall skeleton, allows the pathogen cell wall skeleton generally having a strong fat-soluble ingredient to be easily dispersed in an aqueous solution, thereby preparing various formulations of live pathogen-mimetic nano-molecules using the same. In addition, as various materials capable of inducing cellular immunity are easily included, the pathogen cell wall skeleton induces not only humoral immunity but also cellular immunity, so the limitation of low immunogenicity of existing vaccines can be overcome. In addition, since the present invention does not use a cell itself, stability can increase and mass production is possible. Therefore, by using the pathogen cell wall skeleton-based live pathogen-mimetic nano-molecule of the present invention, it is expected to be effectively used as a vaccine for preventing or treating various diseases such as infectious diseases, tuberculosis, and cancer.

### [Description of Drawings]

FIG. 1 is a schematic diagram illustrating the mechanism of action of a pathogen cell wall skeleton-based nanodispersion according to one embodiment of the present invention.
FIG. 2 is a diagram confirming the degree of stabilization when various types of surfactants and positively-charged buffer solutions were used in the synthesis of a BCG-CWS nanodispersion according to one embodiment of the present invention. A denotes non-detection.
FIG. 3 shows the result of confirming the characteristic of immune cell activation of a BCG-CWS nanodispersion according to one embodiment of the present invention using ELISA.
FIG. 4 shows the result of analyzing CWS-T7/8a according to one embodiment of the present invention using a transmission electron microscope and DLS.
FIG. 5 shows the result of assessing cytokines, using ELISA, secreted when immune cells of each mouse are treated with CWS-T7/8a or components thereof according to one embodiment of the present invention. ND denotes non-detection.
FIG. 6 shows the result of assessing AST and ALT changes in blood over time, using ELISA, after administration of CWS-T7/8a or components thereof according to one embodiment of the present invention to immune cells of each mouse.
FIG. 7 shows the result of confirming the change in antibody titer, using ELISA, after CWS-T7/8a or components thereof according to one embodiment of the present invention are mixed with each antigen and then administered to a mouse.
FIG. 8 shows the result of confirming the degree of activation of cellular immunity in the spleen using a flow cytometer and ELISA after CWS-T7/8a or components thereof according to one embodiment of the present invention are mixed with each antigen and then administered to a mouse.
FIG. 9 shows the result of confirming the degree of activation of antigen-specific T cells in blood, using a flow cytometer, after CWS-T7/8a or components thereof according to one embodiment of the present invention are mixed with each antigen and then administered to a mouse.
FIG. 10 shows the result of confirming the antibody titer in blood and the degree of activation of cellular immunity in the spleen, using ELISA and a flow cytometer and ELISA, respectively, after CWS-T7/8a or components thereof according to one embodiment of the present invention are mixed with a coronavirus (SARS-CoV-2) recombinant protein and then administered to a mouse.
FIG. 11 shows the result of confirming the degree of inhibition of cancer growth and survival rate after CWS-T7/8a or components thereof according to one embodiment of the present invention are mixed with each antibody of cancer cells and then administered to a cancer cell-transplanted mouse.
FIG. 12 shows the result of confirming the degree of inhibition of cancer growth and survival rate after CWS-T7/8a or components thereof according to one embodiment of the present invention are mixed with each antibody of cancer cells and then administered to a cancer cell-transplanted mouse.

### [Modes of the Invention]

A freeze-dried preparation of a pathogen cell wall skeleton, which includes a lipid- and membrane protein-removed pathogen cell wall skeleton of the present invention as an active ingredient, allows a pathogen cell wall skeleton having strong fat solubility to be easily dispersed in an aqueous solution, thereby being able to prepared in various formulations, and easily includes various materials such as materials capable of inducing cellular immunity, immune activation materials and chemotherapeutic agents, thereby preparing nano-molecules such as nanoliposomes, nanomicelles, solid nanoparticles, or nanoemulsions. Therefore, by using the freeze-dried preparation of the pathogen cell wall skeleton including the lipid and membrane protein-removed pathogen cell wall skeleton of the present invention as an active ingredient, various forms of live pathogen-mimetic nano-molecules may be easily prepared, and by using this, it is expected that it can be effectively used as a vaccine for preventing or treating various diseases such as infectious diseases, tuberculosis and cancer.

The "toll-like receptor 7 or 8 agonist-based material" used herein may be selected from the group consisting of imidazoquinoline-, hydroxyadenine-, pteridine-, aminopyrimidine-, benzoazepine-, and thia-oxoguanosine-based compounds as a toll-like receptor 7 or 8 agonist, and the imidazoquinoline-based compounds include type of compounds or pharmaceutically acceptable salts thereof, which are cited in WO 2018 196823, WO 2011 049677, WO 2011 027022, WO 2017 102652, and WO 2019 040491, but the present invention is not limited thereto. In addition, the hydroxyadenine-based compounds include types of compounds or pharmaceutically acceptable salts thereof, cited in WO 2012 080730, WO 2013 068438, WO 2019 036023, WO 2019 035969, WO 2019 035970, WO 2019 035971, WO 2019 035968, CN 108948016, US 2014 8846697, WO 2016 023511, WO 2017 133683, WO 2017 133686, WO 2017 133684, WO 2017 133687, WO 2017 076346, WO 2018 210298, WO 2018 095426, WO 2018 068593, WO 2018 078149, and WO 2018 041763, but the present invention is not limited thereto. The pteridine-based compounds include types of compounds or pharmaceutically acceptable salts thereof, cited in US 2010 0143301, WO 2016 007765, WO 2016 044182, WO 2017 035230, WO 2017 219931, WO 2011 057148, and CN 1087 94486, but the present invention is not limited thereto. The aminopyrimidine-based compounds include types of compounds or pharmaceutically acceptable salts thereof, cited in WO 2010 133885, WO 2012 066335, WO 2012 066336, WO 2012 067268, WO 2013 172479, WO 2012 136834, WO 2014 053516, WO 2014 053595, US 2018 0215720, WO 2012 156498, WO 2014 076221, WO 2016 141092, WO 2018 045144, WO 2015 014815, WO 2018 233648, WO 2014 207082, WO 2014 056593, WO 2018 002319, and WO 2013 117615, but the present invention is not limited thereto. The benzoazepine-based compounds include types of compounds or pharmaceutically acceptable salts thereof, cited in WO 2007 024612, WO 2010 014913, WO 2010 054215, WO 2011 022508, WO 2011 022509, WO 2012 097177, WO 2012 097173, WO 2016 096778, WO 2016 142250, WO 2017 202704, WO 2017 202703, WO 2017 216054, WO 2017 046112, and WO 2017 197624, but the present invention is not limited thereto. The thia-oxoguanosine-based compounds include types of compounds or pharmaceutically acceptable salts thereof, cited in WO 2016 180691, WO 2016 055553, WO 2016 180743, and WO 2016 091698, but the present invention is not limited thereto. In addition, the toll-like receptor 7 or 8 compounds or pharmaceutically acceptable salts thereof, cited in PCT/US2009/035563, PCT/US2015/028264, PCT/US2016/020499, WO 2015 023598, and PCT/US 2015/039776, may be included, but the present invention is not limited thereto. All toll-like receptor 7 or 8 agonists, for example, imiquimod, resiquimod, dactolisib, gardiquimod, sumanirole, motolimod, vesatolimod, loxoribine, SM360320, CL264, 3M-003, IMDQ, and Compound 54, which can be easily suggested and used by a person skilled in the art, are included.

In the specification, various toll-like receptor 7 or 8 agonists in which a lipid is conjugated at an activation site may be prepared by the method disclosed in Korean Publication Patent No. 10-2020-0097656, and more specifically, they are prepared by binding of a lipid component such as cholesterol to an amine (NH₂) site, which is the activation site of the toll-like receptor 7 or 8 agonist through one or more selected from the group consisting of a carbamate bond, a disulfide bond, an ester bond, a peptide bond, an azide bond, an amide bond, a hydrazone bond, a thioether bond, a phosphodiester bond, a thioketal bond, and a combination thereof. Representative examples thereof include a carbamate bond (Scheme 1) and a disulfide bond (Scheme 2).

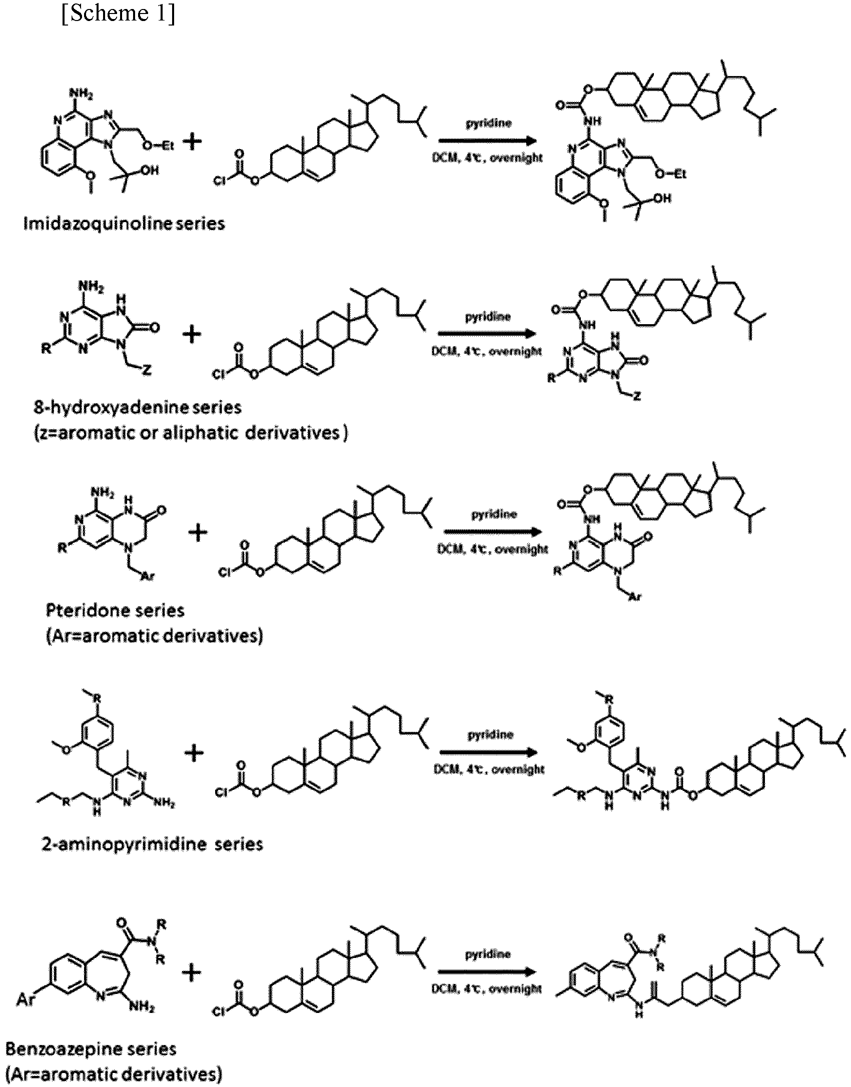

R is a branch having an aliphatic or aromatic group, and may include -NH-, - CO-. -CONH-, -CSNH-, -COO-, -CSO-, -SO₂NH-, -SO₂-, -SO-, or -O-.

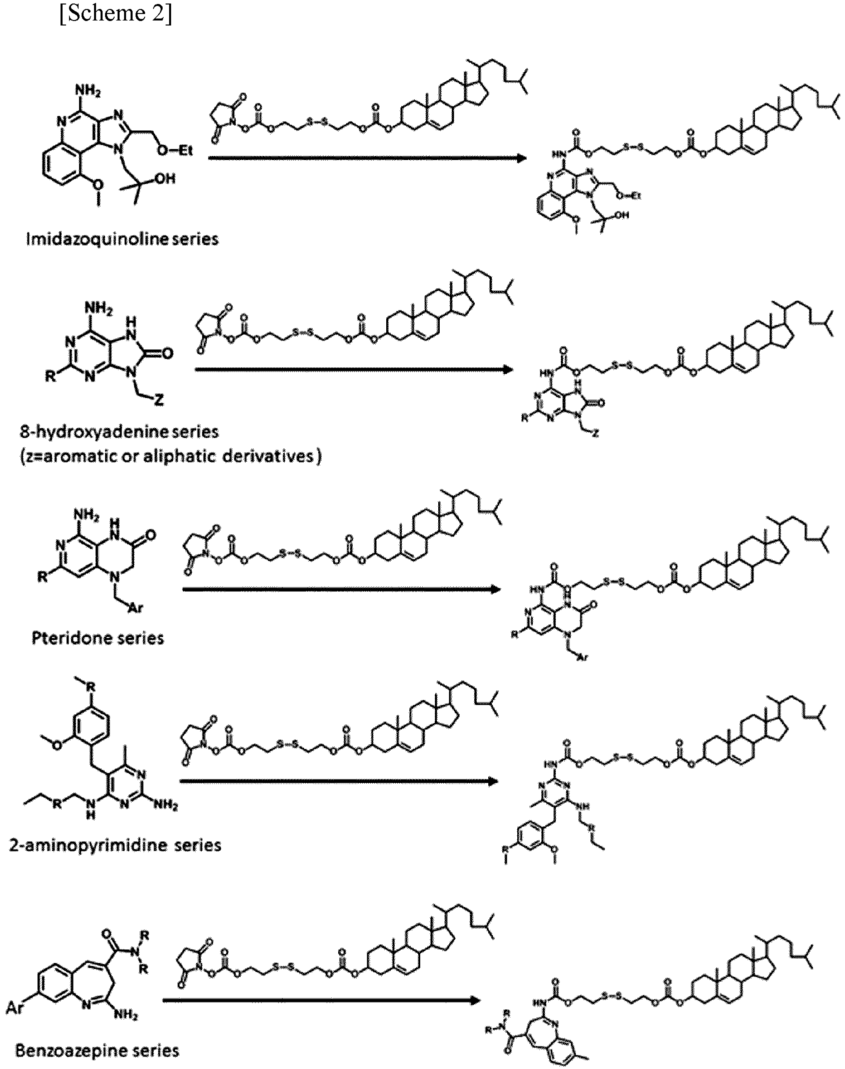

R is a branch having an aliphatic or aromatic group, and may include -NH-, - CO-, -CONH-, -CSNH-, -COO-, -CSO-, -SO₂NH-, -SO₂-, -SO-, or -O-.

The "toll-like receptor 7 or 8 agonist-based material" used herein includes single stranded ribonucleic acids (ssRNAs), guanosine (G)-rich, uridine (U)-rich and adenosine (A)-rich oligonucleotides, and natural ligands such as small-interfering RNA (siRNA) and microRNA (miRNA) as a toll-like receptor 7 or 8 agonist.

The "toll-like receptor agonist" used herein may be one that causes a signaling response via TLR-1, and in one example, any one or more materials selected from the group consisting of tri-acylated lipopeptide (LP); phenol-soluble modulin; mycobacterium tuberculosis lipopeptide; S-(2,3-bis(palmitoyloxy)-(2-RS)-propyl)-N-palmitoyl-(R)-Cys-(S)-Ser-(S)-Lys(4)-OH; *Borrelia burgdorfei*-derived lipopeptide; trihydrochloride (Pam3Cys) lipopeptide mimicking the acetylated amino terminus of OspA lipopeptide; and a combination thereof. In addition, the toll-like receptor agonist may be the TLR-2 agonist such as Pam3Cys-Lip, and the TLR-3 agonists such as poly(I:C), poly(ICLC), poly(IC12U), and ampligen. In addition, the toll-like receptor agonist may be a *Shigella flexineri* outer membrane protein construct, or a TLR-4 agonist such as AGP, CRX-527, MPLA, PHAD, 3D-PHAD or GLA, or a TLR-5 agonist such as flagellin or a flagellin fragment. In addition, the toll-like receptor agonist may be a TLR-9 agonist such as an immunostimulatory oligonucleotide. However, the present invention is not limited thereto, and any toll-like receptor agonist that can be easily suggested and used by a person in the art skilled in the art may be included.

The "saponin" used herein may be an amphipathic glycoside present in various plant species, and for example, QS21, Quil A, QS7, QS17, β-eskin, digitonin, or a combination thereof, but the present invention is not limited thereto.

The "antiviral peptide" used herein may be a peptide that can inhibit viral proliferation, and for example, KLK, but the present invention is not limited thereto.

The "inflammasome inducer" used herein may be trehalose-6,6-dibehenate (TDB), NLRC4, or Nalp3, but the present invention is not limited thereto.

The "NOD ligand" used herein may be NOD2 agonist-synthetic muramyl tripeptide (M-TriLYS), an NOD2 agonist (N-glycolylated muramyldipeptide), mDAP, or MDP, but the present invention is not limited thereto.

The "CDS ligand" used herein may be poly(dA:dT), but the present invention is not limited thereto.

The "STING ligand" used herein may be cGAMP, di-AMP, or di-GMP, but the present invention is not limited thereto.

The "lipid" used herein may be fatty acids, steroids, glycerides, phospholipids, cholesterols, or fat-soluble vitamins, but the present invention is not limited thereto. In addition, the "lipid complex" used herein encompasses all compounds bound with the lipid.

In addition, the materials that may be further included may be composite immunomodulatory substances, and for example, CL401 (TLR-2 and TLR-7 agonists) or CL429 (TLR-2 and NOD2 agonists), but the present invention is not limited thereto.

In the specification, the chemotherapeutic agent has no limitation as long as it is a compound that is used in cancer treatment, which is known to those of ordinary skill in the art, and examples thereof include paclitaxel, docetaxel, 5-flurouracil, alendronate, doxorubicin, simvastatin, hydrazinocurcumin, amphotericin B, ciprofloxacin, rifabutin, rifampicin, efavirenz, cisplatin, theophyline, pseudomonas exotoxin A, zoledronic acid, trabectedin, siltuximab, dasatinib, sunitinib, apatinib, 5,6-dimethylxanthenone-4-acetic acid, silibinin, PF-04136309, trabectedin, carlumab, BLZ945, PLX3397, emactuzumab, AMG-820, IMC-CS4, GW3580, PLX6134, N-acetyl-l-cysteine, vitamin C, bortezomib, aspirin, salicylates, indolecarboxamide derivatives, quinazoline analogues, thalidomide, prostaglandin metabolites, 2ME2, 17-AAG, camptothecin, topotecan, pleurotin, 1-methylpropyl, 2-imidazolyl disulfide, tadalafil, sildenafil, L-AME, nitroaspirin, celecoxib, NOHA, bardoxolone methyl, D,L-1-methyl-tryptophan, gemcitabine, axitinib, sorafenib, cucurbitacin B, JSI-124, anti-IL-17 antibodies, anti-glycan antibodies, anti-VEGF antibodies, bevacizumab, antracycline, tasquinimod, imatinib or cyclophosphamide, but the present invention is not limited thereto.

The "immune checkpoint inhibitor" used herein is the generic term for cancer therapies which activate the immune function of immune cells of the human body to make them fight cancer cells, and is, for example, anti-PD-1, anti-PD-L1, anti-CTLA-4, anti-KIR, anti-LAG3, anti-CD137, anti-OX40, anti-CD276, anti-CD27, anti-GITR, anti-TIM3, anti-41BB, anti-CD226, anti-CD40, anti-CD70, anti-ICOS, anti-CD40L, anti-BTLA, anti-TCR, or anti-TIGIT, but the present invention is not limited thereto.

The "antigen" used herein encompasses all substances causing an immune response in the body, and preferably, pathogens (bacteria, viruses, etc.), compounds, pollen, cancer cells, shrimp, or some peptides or proteins thereof, and more preferably, a cancer antigen peptide. However, there is no limitation as long as it is a substance that can cause an immune response in the body. The antigen is preferably a protein, a recombinant protein, a glycoprotein, a gene, a peptide, a polysaccharide, a lipopolysaccharide, a polynucleotide, cells, a cell lysate, bacteria or viruses, and more preferably, a cancer antigen peptide. The protein may be an antibody, an antibody fragment, a structural protein, a regulatory protein, a transcription factor, a toxin protein, a hormone, a hormone analogue, an enzyme, an enzyme fragment, a transport protein, a receptor, a receptor fragment, a biodefense inducer, a storage protein, a movement protein, an exploitive protein, or a reporter protein. However, there is no limitation as long as it is a material that acts as an antigen in a living organism and can induce an immune response.

The "vaccine" used herein is a biological preparation containing an antigen causing an immune response in a living organism, and refers to an immunogen that generates immunity in the living organism by injection or oral administration into a human or animal to prevent or treat a disease such as cancer, tuberculosis or an infectious disease. The animal is a human or a non-human animal, and the non-human animal refers to, but is not limited to, a pig, a cow, a horse, a dog, a goat, a sheep, or the like.

The "prevention" used herein refers to all actions of inhibiting an infectious disease, tuberculosis or cancer or delaying the onset thereof by administration of the composition according to the present invention.

The term "treatment" used herein refers to all actions involved in alleviating or beneficially changing symptoms of an infectious disease, tuberculosis or cancer by administration of the composition according to the present invention.

The "subject" used herein refers to a subject into which the composition of the present invention can be administered, and there is no limitation to the subject.

The "cancer" used herein is the generic term for various hematological cancers and malignant solid tumors that can expand locally by infiltration and systemically through metastasis. While not particularly limited, specific examples of cancer include colorectal cancer, adrenal cancer, bone cancer, brain cancer, breast cancer, bronchial cancer, colon cancer and/or rectal cancer, gallbladder cancer, gastrointestinal cancer, head and neck cancer, kidney cancer, laryngeal cancer, liver cancer, lung cancer, nerve tissue cancer, pancreatic cancer, prostate cancer, parathyroid cancer, skin cancer, stomach cancer, and thyroid cancer. Other examples of the cancer include adenocarcinoma, adenoma, basal cell carcinoma, cervical dysplasia and in situ carcinoma, Ewing's sarcoma, epidermoid carcinomas, giant cell tumors, glioblastoma multiforma, hairy-cell tumors, intestinal ganglioneuroma, hyperplastic corneal nerve tumors, islet cell carcinoma, Kaposi's sarcoma, leiomyoma, leukemia, lymphoma, malignant carcinoid, malignant melanoma, malignant hypercalcemia, marfanoidhabitus tumors, medullary carcinoma, metastatic skin carcinoma, mucosal neuroma, myelodisplastic syndrome, myeloma, mycosis fungoides, neuroblastoma, osteosarcoma, osteogenic and other sarcomas, ovarian tumors, pheochromocytoma, polycythermia vera, primary brain tumors, small-cell lung tumors, squamous cell carcinoma of both ulcerating and papillary types, seminoma, soft tissue sarcoma, retinoblastoma, rhabdomyosarcoma, renal cell tumors or renal cell carcinoma, veticulum cell sarcoma, and Wilm's tumors. In addition, the cancer includes astrocytoma, gastrointestinal stromal tumor (GIST), glioma or glioblastoma, renal cell carcinoma (RCC), hepatocellular carcinoma (HCC), and pancreatic neuroendocrine tumors.

The "vaccine composition" used herein may be in the form of a capsule, a tablet, a granule, an injection, an ointment, a power or a drink, and the vaccine composition is for humans. The vaccine composition may be, but is not limited to, formulated in the form of an oral formulation such as a powder, granules, a capsule, a tablet or an aqueous suspension, a preparation for external use, a suppository and a sterile injectable solution according to a conventional method. The vaccine composition of the present invention may include a pharmaceutically acceptable carrier. As pharmaceutically acceptable carriers, a binder, a lubricant, a disintegrant, an excipient, a solubilizer, a dispersant, a stabilizer, a suspending agent, a coloring agent and a flavor may be used for oral administration, a mixture of a buffer, a preservative, a pain relief agent, a solubilizer, an isotonic agent and a stabilizer may be used for an injectable, and a base, an excipient, a lubricant and a preservative may be used for local administration. The vaccine composition of the present invention may be prepared in various forms by being mixed with the above-described pharmaceutically acceptable carrier. For example, for oral administration, the vaccine composition of the present invention may be prepared in various dosage forms such as a tablet, a troche, a capsule, an elixir, a suspension, a syrup and a wafer, and for injectables, the vaccine composition of the present invention may be prepared in a unit dose ampoule or multiple dose forms. In addition, the vaccine composition of the present invention may be formulated as a solution, a suspension, a tablet, a capsule or a sustained-release preparation.

Meanwhile, examples of carriers, excipients and diluents suitable for preparation may include lactose, dextrose, sucrose, sorbitol, mannitol, xylitol, erythritol, malditiol, starch, acacia gum, alginate, gelatin, calcium phosphate, calcium silicate, cellulose, methyl cellulose, microcrystalline cellulose, polyvinylpyrrolidone, water, methylhydroxybenzoate, propylhydroxybenzoate, talc, magnesium stearate, and mineral oil. In addition, another examples of carriers, excipients and diluents may include a filler, an anti-agglomerate, a lubricant, a wetting agent, a fragrance, an emulsifier, and a preservative.

Administration routes for the vaccine composition according to the present invention may include, but are not limited to, oral, intravenous, intramuscular, intraarterial, intramedullary, intrathecal, intracardiac, transdermal, subcutaneous, intraperitoneal, intranasal, enteral, local, sublingual or rectal administration. Oral or parenteral administration is preferable. The term "parenteral" used herein means subcutaneous, intracutaneous, intravenous, intramuscular, intraarticular, intrabursal, intrasternal, intrathecal, intralesional and intracranial injections, or infusions. The vaccine composition of the present invention may also be administered in the form of a suppository for rectal administration.

A dose of the vaccine composition of the present invention may vary according to various factors including the activity of a specific compound used, age, body weight, general health, sex, diet, administration time, administration route, excretion rate, drug formulation, and the severity of a specific disease to be prevented or treated, and a dosage of the vaccine composition may be suitably selected by those of ordinary skill in the art depending on a patient's condition, body weight, the severity of a disease, a drug type, an administration route and an administration duration, and may be 0.0001 to 500 mg/kg or 0.001 to 500 mg/kg per day. The vaccine composition of the present invention may be administered once a day or several times in divided portions. The dose does not limit the scope of the present invention in any way. The vaccine composition according to the present invention may be formulated as a pill, a sugar-coated tablet, a capsule, a liquid, a gel, a syrup, a slurry or a suspension.

In addition, the vaccine composition according to the present invention may further include a conventionally known "adjuvant." The adjuvant generally refers to any substance that increases humoral and/or cellular immune response(s) to an antigen, and there is no limitation as long as it is one that is known in the art. For example, Freund's complete or incomplete adjuvant may be further included to increase immunity. In addition, in the case of the vaccine composition, if necessary, the stimulation with an antigen at an initial dose and optionally repeated doses may be performed.

Hereinafter, to help in understanding the present invention, exemplary examples will be suggested. However, the following examples are merely provided to more easily understand the present invention, and not to limit the present invention.

### [Examples]

### Example 1: Preparation of pathogen outer cell wall component-based nanodispersion

To confirm whether a nanodispersion based on a pathogen cell wall skeleton (CWS) is prepared, first, a nanodispersion was prepared using a Bacilli-Galmette-Guerin (BCG) strain (CAVAC). In further detail, after dispersing the strain in phosphate buffered saline (PBS), the cells were disrupted using a sonicator (Sonics & Materials). Then, after centrifugation at 1000x g for 3 minutes to obtain disrupted cells, a 1% Triton X-100 solution was added to the disrupted cells, and lipids were isolated by sonication at 90 °C using a sonicator (Emerson) for 2 hours while heating in hot water. Then, the supernatant was removed by centrifugation at 10,000x g, and 1% SDS was added to a pellet, and the membrane proteins were separated by applying ultrasonic waves at 90 °C for 2 hours using a sonicator (Emerson) and heating in hot water. Afterward, by centrifugation at 10,000x g and removal of a supernatant, a BCG-outer cell wall component (BCG-CWS) from which lipids and membrane protein components were removed was acquired. The acquired BCG-CWS was redispersed using ethanol and then rapidly frozen using liquid nitrogen. Then, the frozen cells were treated using a freeze-dryer to remove ethanol, thereby preparing a powder form. Then, the powder-type BCG-CWS was added to a 40 mM L-(+)-lysine solution to have a concentration of 1 mg/mL, and dispersed using a sonicator at 60 °C for 24 hours, thereby preparing a BCG-CWS nanodispersion. The mechanism of action of the pathogen cell wall skeleton-based nanodispersion of the present invention is schematically shown in FIG. 1.

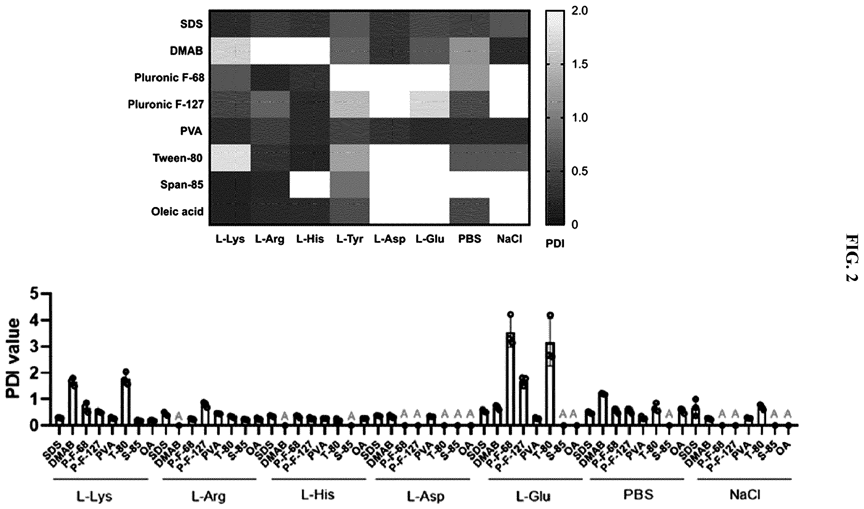

Generally, since the outer wall component of BCG is dispersed in an organic phase due to strong hydrophobicity, to confirm whether the BCG-CWS prepared by the method of the present invention is easily dispersed in various surfactants and positively-charged buffer solutions (buffers), the BCG-CWS nanodispersion was added to each of a surfactant such as sodium dodecyl sulfate (SDS), Tween, Pluronic F, polyvinyl alcohol (PVA), Span, or oleic acid and various types of positively-charged buffer solutions, and a polydispersity index (PDI) was measured. The result is shown in FIG. 2.

As shown in FIG. 2, it was confirmed that the BCG-CWS nanodispersion is easily dispersed in various surfactants and positively-charged buffer solutions and exhibits stability. From the above result, it can be confirmed that, according to the method of the present invention, various pathogen outer wall component-based nanodispersions, which are easily dispersed in an aqueous solution, can be prepared.

### Example 2: Confirmation of immune cell activation characteristic of pathogen cell wall skeleton-based nanodispersion

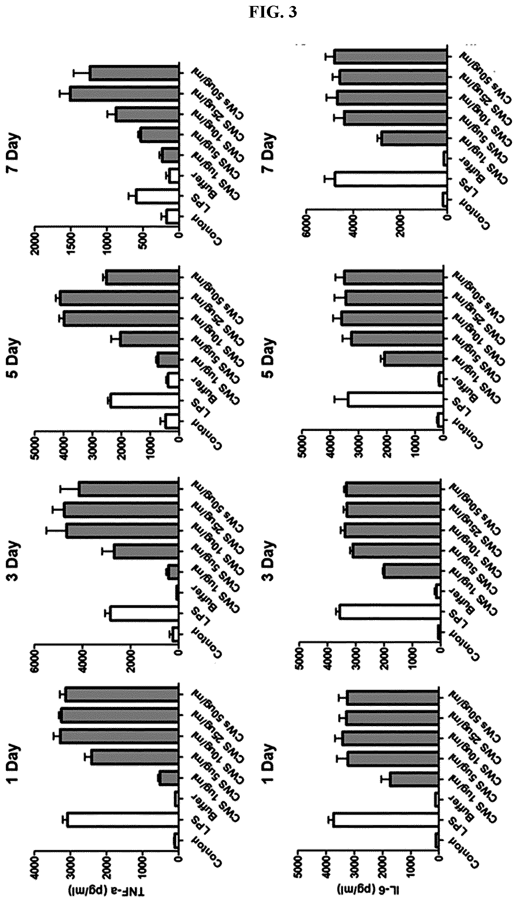

To confirm whether the BCG-CWS nanodispersion prepared in the same manner as described in Example 1 has the characteristic of immune cell activation, bone marrow-derived dendritic cells (BMDCs) were treated with the BCG-CWS nanodispersion at a concentration of 1, 5, 10, 25, and 50 µg/mL. Then, the cells were cultured for 7 days, and the secretion amounts of tumor necrosis factor-a (TNF-α) and interleukin-6 (IL-6), which are representative indicators of a inflammatory response, were assessed using an ELISA kit. As a positive control, lipopolysaccharide (LPS) known to induce an inflammatory response was treated and compared. The result is shown in FIG. 3.

As shown in FIG. 3, it was confirmed that the secretion amounts of TNF-α and IL-6 are increased in BCG-CWS nanodispersion-treated bone marrow-derived dendritic cells, and thereby, it was confirmed that the pathogen cell wall skeleton-based nanodispersion prepared by the method of the present invention effectively activates immune cells. In addition, it was able to be confirmed that the pathogen cell wall skeleton-based nanodispersion prepared by the method of the present invention can be used as live pathogen-mimetic nano-molecules.

### Example 3: Preparation of live pathogen-mimetic nano-molecule using pathogen cell wall skeleton-based nanodispersion

### 3.1. Synthesis of conjugate of toll-like receptor 7 or 8 agonist and cholesterol

50 g (0.5 molar equivalent, AmBeed, Inc. USA) of bis(2,5-dioxopyrrolidin-1-yl)(disulfanediylbis(ethane-2,1-diyl))dicarbonate) and 1 L of dichloromethane (DCM, SIGMA) were added to a reactor at room temperature and mixed. 49 g (0.55 molar equivalent, AVANTI Polar lipid) of cholesterol and 23 g (1.0 molar equivalent) of triethylamine (TEA, SIGMA) were added to the mixed solution and stirred at room temperature for 3 hours, and then 1.5 L distilled water was added. An aqueous layer was extracted from the solution having completed mixing using 500 mL of DCM. To obtain high purity, the extraction procedure was repeated three times. Then, an organic layer included in the solution having completed extraction was removed using 1 L of brine (high concentration of brine) and dried with 100 g of anhydrous sodium sulfate (Na₂SO₄, SIGMA) under reduced pressure. The dried material was added to a 300 g silica gel-filled column for chromatography and extracted with a 5 to 20% EtOAc-added n-hexane solution, thereby preparing a cholesterol compound. Afterward, to bind a toll-like receptor 7 or 8 agonist (TLR 7/8 agonist) with cholesterol, 7.0 g resiquimod, which is one of the toll-like receptor 7 or 8 agonists, and 6.5 g of DCM, 0.6 g of a cholesterol compound, and 0.3 g of TEA were added to the reactor at room temperature and stirred for 10 hours or more. Afterward, distilled water was added so as to be 20 times the total volume of the solution, and the aqueous layer was extracted five or more times using 3.3 g of DCM. Then, an organic layer included in the solution having completed extraction was removed using 1 L of brine and dried with 7 g of anhydrous Na₂SO₄ under reduced pressure. The dried material was added to a 100 g of silica gel-filled column for chromatography and extracted with a 10 to 50 % EtOAc-added n-hexane solution, thereby synthesizing a complex of the toll-like receptor 7 or 8 agonist and cholesterol, which was named T7/8a.

### 3.2. Preparation of live pathogen-mimetic nano-molecule including BCG-CWS nanodispersion and toll-like receptor 7 or 8 agonist

To prepare a live pathogen-mimetic nano-molecule using a BCG-CWS nanodispersion and a toll-like receptor 7 or 8 agonist, a BCG-CWS nanodispersion prepared in the same manner as in Example 1 and T7/8a was added to a chloroform solution in a 1 : 1 ratio (w:w) and dissolved, the resulting solution was transferred to a round-bottom flask, and an organic solvent was completely evaporated to form a film using a rotary evaporator. Then, a Span-85-added 40 mM L-(+)-lysine solution was added to the flask so that a final concentration was 1% and the concentration of the BCG-CWS nanodispersion became 1 mg/mL, and completely dispersed by stirring at 60 °C and 300 rpm for 1 hour. Then, the resulting mixture was stabilized for 1 hour, thereby preparing a live pathogen-mimetic nano-molecule including the BCG-CWS nanodispersion and the toll-like receptor 7 or 8 agonist, which was named CWS-T7/8a.

### 3.3. Confirmation of characteristics of live pathogen-mimetic nano-molecule

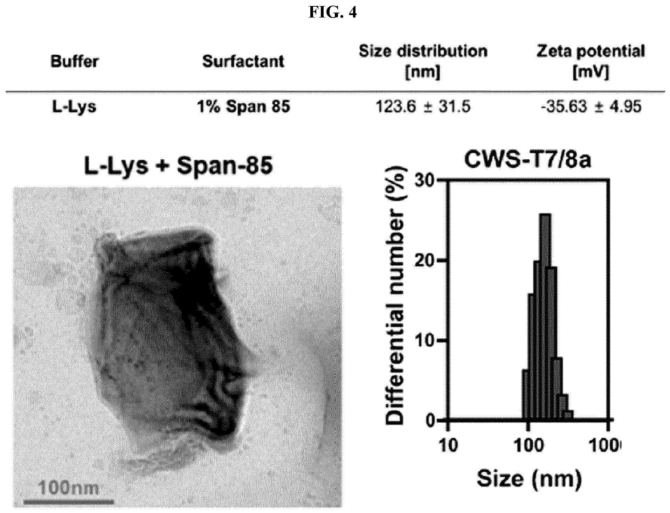

The characteristics of the live pathogen-mimetic nano-molecule prepared in the same manner as in Example 3.2 were confirmed using dynamic light scattering (DLS) and an electron microscope. The result is shown in FIG. 4.

As shown in FIG. 4, it was confirmed that the live pathogen-mimetic nano-molecule has an average diameter of approximately 120 nm, and exhibits a negative charge.

### 3.4. Confirmation of immune response activation effect of live pathogen-mimetic nano-molecule

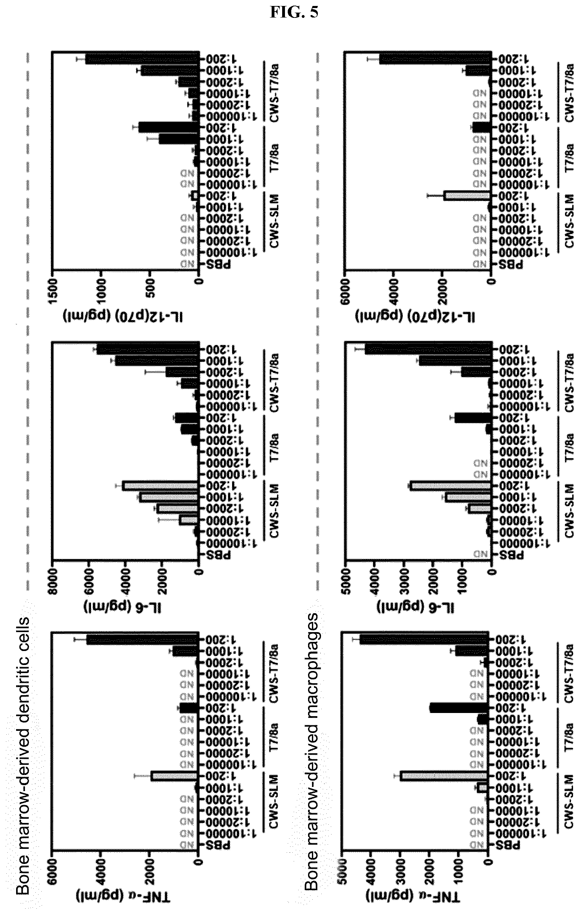

To confirm whether the live pathogen-mimetic nano-molecule prepared in the same manner as in Example 3.2 activates an immune response, bone marrow-derived dendritic cells and bone marrow-derived macrophages (BMDMs) were treated with a CWS nanodispersion (CWS-SLM), T7/8a, or a live pathogen-mimetic nano-molecule (CWS-T7/8a) diluted to different concentrations. Then, the cells were cultured for 7 days and the secretion amounts of TNF-α, IL-6, and IL-12, which are representative indictors of an inflammatory response, were confirmed using an ELISA kit. Afterward, all tests were repeated at least three times, and the result was expressed as mean ± standard deviation. Statistical significance was confirmed by a Student's t-test, and ^{∗} denotes P < 0.05, ^{∗∗} denotes P < 0.01, ^{∗∗∗} denotes P < 0.001, and ^{∗∗∗∗} denotes P < 0.0001. When P < 0.05, it was determined to be statistically significant. The result is shown in FIG. 5.

As shown in FIG. 5, it was confirmed that, not only the secretion of cytokines involved in an inflammatory immune response in immune cells, TNF-α and IL-6, is greatly increased, but the secretion of the important indicator for a cellular immune response, that is, IL-12 (p70), is also increased. From the above result, it was able to be confirmed that the live pathogen-mimetic nano-molecule of the present invention primarily activates a pathogen recognition-mediated cellular immune response based on a pathogen cell wall skeleton and is secondarily delivered into the immune cells through phagocytosis, and cytokines involved in the inflammatory immune response are secreted from immune cells through the action of activating signal transduction and a cellular immune response by separating a toll-like receptor 7 or 8 agonist associated with a live/death signal(s) from cholesterol in an endo-lysosome (FIG. 1).

### 3.5. Evaluation of toxicity of live pathogen-mimetic nano-molecule

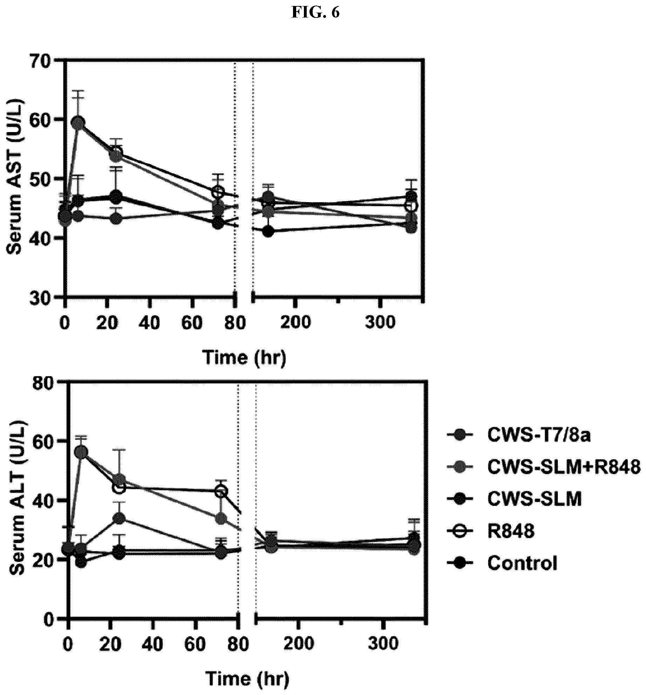

To evaluate the toxicity of a live pathogen-mimetic nano-molecule, a live pathogen-mimetic nano-molecule, a CWS-nanodispersion, resiquimod (R848; free drug), or a mixture of a CWS-nanodispersion and resiquimod (R848; free drug), which was added to a 50 µL of phosphate buffered saline (PBS) to have a resiquimod concentration of 25 µg, was subcutaneously injected into the right flank of a C57BL/6 mouse. Then, blood was extracted from the mouse at fixed time intervals and centrifuged at 12,000 rpm to isolate plasma. Then, the concentrations of aspartate transaminase (AST) and alanine transaminase (ALT) contained in the isolated plasma were measured using ELISA. The result is shown in FIG. 6.

As shown in FIG. 6, it was confirmed that, in the experimental group treated with resiquimod as a free drug, AST and ALT, which are indicators of hepatotoxicity, were initially increased, but in the case of the CWS-nanodispersion and the live pathogen-mimetic nano-molecule, the concentrations of AST and ALT were hardly changed.

From the above results, it was able to be confirmed that the live pathogen-mimetic nano-molecule of the present invention exhibits little toxicity in the body, and not only effectively induces an inflammatory immune response in immune cells, but also increases the cellular immune response, and therefore, it was able to be confirmed that the live pathogen-mimetic nano-molecule of the present invention can be used as an effective immune activator.

### Example 4: Preparation and application of vaccine using live pathogen-mimetic nano-molecule

### 4.1. Preparation of vaccine using live pathogen-mimetic nano-molecule

To use the live pathogen-mimetic nano-molecule prepared in the same manner as in Example 3 as a vaccine, 0.2 mg/mL of ovalbumin (OVA), which is an antigen protein, was mixed with the live pathogen-mimetic nano-molecule in a 1:1 volume ratio, and then stirred using a stirrer at 4 °C and 300 rpm for 30 minutes to allow the live pathogen-mimetic nano-molecule to interact with the antigen. A control was prepared by reacting liposomes (T7/8a liposomes) including each of CWS-nanodispersion, resiquimod, and a cholesterol-resiquimod complex were used alone or in combination with each other, with the antigen protein.

### 4.2. Confirmation of antibody production efficacy of vaccine using live pathogen-mimetic nano-molecule

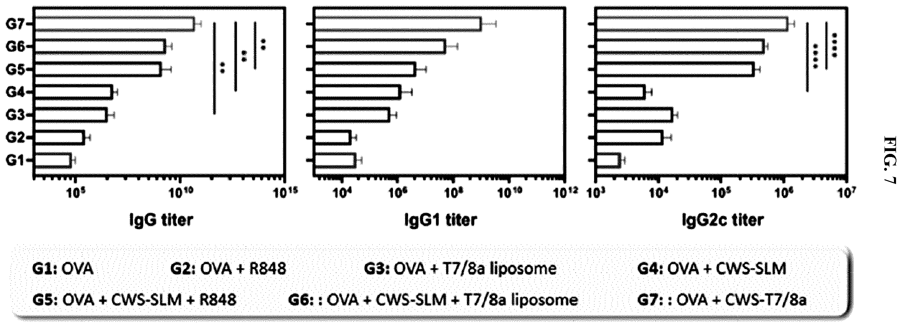

To confirm the antigenicity and efficacy of the vaccine prepared in the same manner as in Example 4.1, the vaccine was injected into a C57BL/6 mouse twice at 100 µL every two weeks. Then, one week after the last injection, blood and the spleen were extracted from the mouse. Then, the extracted blood was centrifuged at 12,000 rpm to isolate plasma, and an antibody titer (IgG titer) was measured. The result is shown in FIG. 7.

As shown in FIG. 7, it was confirmed that the vaccine prepared using the live pathogen-mimetic nano-molecule of the present invention exhibits a high antibody titer at all indicators compared to an antigen alone or other controls.

### 4.3. Confirmation of T-cell activation efficacy of vaccine using live pathogen-mimetic nano-molecule

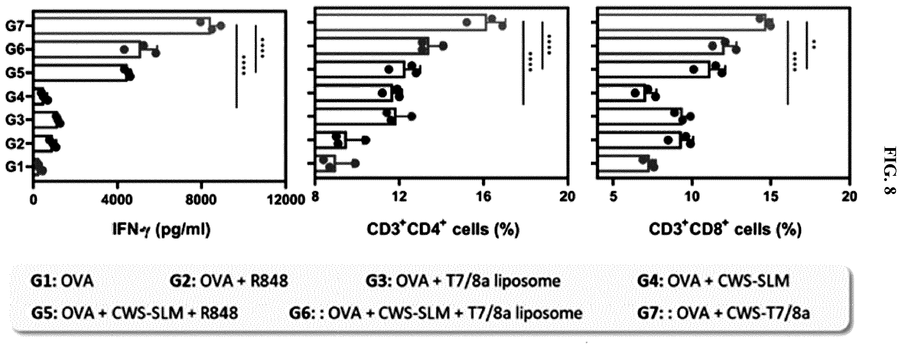

To confirm the T-cell activation efficacy of a vaccine using a live pathogen-mimetic nano-molecule, splenocytes were extracted from the spleen extracted in the same manner as in Example 4.2 and seeded and cultured in a 6-well plate (Corning Inc.) to become 1×10⁶ cells. Then, after adding 40 µg of OVA, the cultured splenocytes were incubated for 2 days. Then, the supernatant of the cell culture was obtained, the concentration of IFNγ was measured using ELISA, and CD4⁺ T cells and CD8⁺ T cells were counted by a flow cytometer (FACS). The result is shown in FIG. 8.

As shown in FIG. 8, the vaccine prepared using a live pathogen-mimetic nano-molecule shows the highest amount of secreted IFNγ and highest proportions of CD4⁺ T cells and CD8⁺ T cells compared to an antigen alone or other controls.

### 4.4. Confirmation of antigen-specific T cell production efficacy of vaccine using live pathogen-mimetic nano-molecule

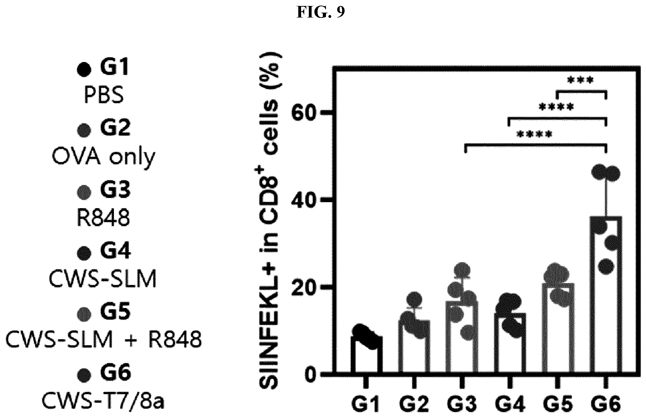

To confirm antigen-specific T cell production efficacy, peripheral blood monocytic cells (PBMCs) were isolated from the blood extracted in the same manner as in Example 4.2 using Percoll^{®}. Then, the PBMCs were treated with a fluorescence antibody reacting with SIINFEKL known as an action site of OVA, and confirmed using a flow cytometer. The result is shown in FIG. 9.

As shown in FIG. 9, the vaccine prepared using a live pathogen-mimetic nano-molecule of the present invention shows a significantly high proportion of antigen-specific T cells in the blood compared with an antigen alone or other controls.

### 4.5. Preparation of corona vaccine using live pathogen-mimetic nano-molecule and confirmation of its efficacy

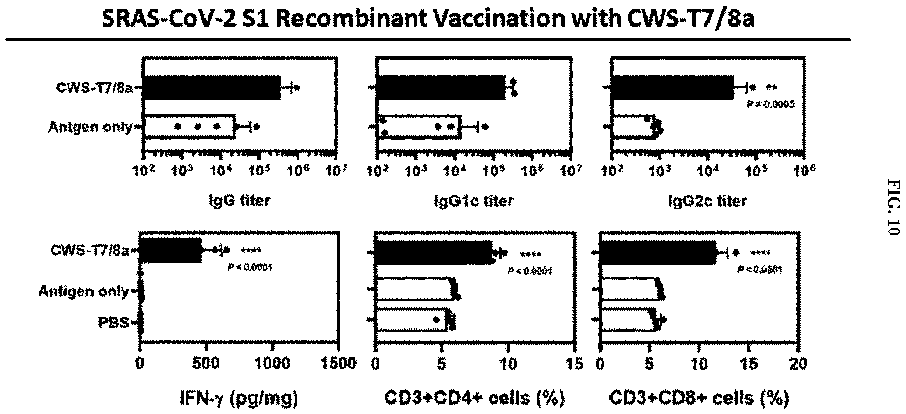

To prepare a corona vaccine using a live pathogen-mimetic nano-molecule, a recombinant S1 protein (Arigo) of SARS-CoV-2 was mixed with a live pathogen-mimetic nano-molecule at a 1:1 volume ratio and stirred at 4 °C and 300 rpm for 30 minutes to allow the live pathogen-mimetic nano-molecule to interact with the antigen, thereby preparing a vaccine. Then, the prepared vaccine was injected into a C57BL/6 mouse twice at 100 µL every two weeks. One week after the final injection, blood and the spleen were extracted from the mouse, and vaccine efficacy was confirmed by the same manner as in Examples 4.2 and 4.3. The result is shown in FIG. 10.

As shown in FIG. 10, compared to a control administered an antigen alone, it was confirmed that all indicators show high antibody titers. In addition, in the control administered an antigen alone, an amount of secreted IFNγ and the proportions of CD4⁺ T cells and CD8⁺ T cells, which are similar to those of PBS, were shown, but in the case of the corona vaccine using a live pathogen-mimetic nano-molecule, it was confirmed that the amount of secreted IFNγ and the proportions of CD4⁺ T cells and CD8⁺ T cells were all increased.

From the above results, a vaccine using a live pathogen-mimetic nano-molecule of the present invention can significantly increase the proportion and antibody titer of T cells, which are immune cells, compared with a vaccine using an antigen alone, and thus it was able to be confirmed that vaccine efficacy could significantly increase by applying the live pathogen-mimetic nano-molecule of the present invention to various existing vaccines.

### Example 5: Confirmation of anticancer immune therapy effect using live pathogen-mimetic nano-molecule

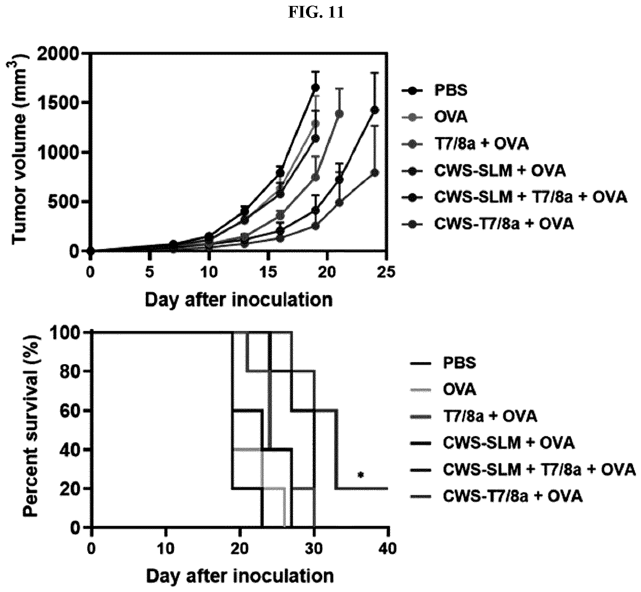

To confirm the cancer prevention efficacy of the vaccine using a live pathogen-mimetic nano-molecule, a vaccine prepared in the same manner as in Example 4.1 was injected into a C57BL/6 mouse twice at 100 µL every week. Then, after one week, 5×10⁵ B16-OVA cancer cells were subcutaneously injected into a C57/BL6 mouse to cause cancer, and cancer growth and a survival rate were confirmed. The result is shown in FIG. 11.

As shown in FIG. 11, in the case of the vaccinated experimental group, it was confirmed that the cancer growth was inhibited, and the survival rate was also significantly increased.

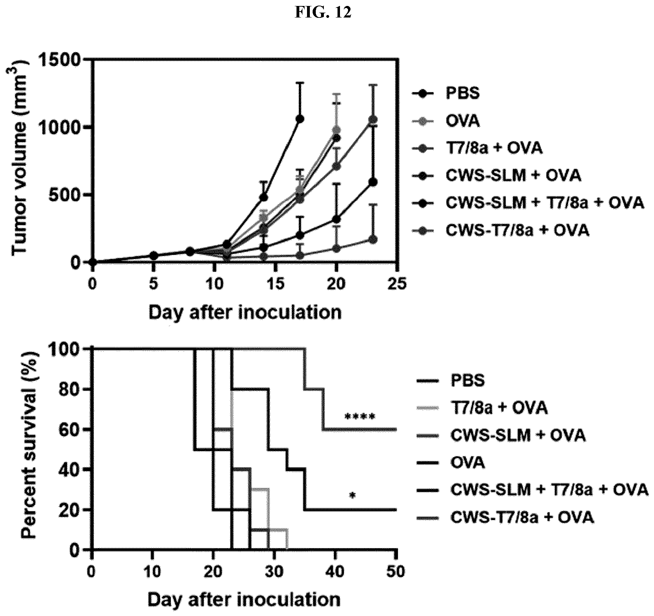

In addition, to confirm the cancer treatment efficacy of the vaccine using a live pathogen-mimetic nano-molecule, 5×10⁵ B16-OVA cancer cells were subcutaneously injected into a C57BL/6 mouse to cause cancer, and when the cancer size reached 50 mm³, a vaccine prepared in the same manner as in Example 4.1 was injected twice at 100 µL every week. Then, cancer growth and a survival rate were confirmed. The cancer growth was measured with the increased cancer size at 50 mm³. The result is shown in FIG. 12.

As shown in FIG. 12, in the case of the vaccinated experimental group, it was confirmed cancer growth was inhibited and the survival rate was also significantly increased.

From the above results, it was able to be confirmed that the vaccine using a live pathogen-mimetic nano-molecule of the present invention exhibits a cancer prevention and treatment effect, and therefore a vaccine using a live pathogen-mimetic nano-molecule of the present invention can be used as an immunotherapeutic agent.

Generally, while the pathogen cell wall skeleton is dispersed only in an organic phase due to strong hydrophobicity, since the nanodispersion based on the pathogen cell wall skeleton prepared by the method of the present invention is easily dispersed in an aqueous solution without using an fat-soluble solvent, it was able to be confirmed that it can be easily prepared in various types of nano-molecules such as a nanoliposome, a nanoemulsion, a nanomicelle, and a polymer nanoparticle. In addition, from the above results, it was able to be confirmed that the nanodispersion based on the pathogen cell wall skeleton prepared by the method of the present invention maintains the immune cell activation efficacy of a pathogen itself, so it can be used not only to prepare various pharmaceuticals, but also widely applied to various diseases as an immune activation therapeutic agent. In addition, by including various adjuvants in the nanodispersion based on the pathogen cell wall skeleton of the present invention, it was confirmed that not only humoral immunity but also cellular immunity can be effectively induced, and the immunogenic effect can also be further improved.

It should be understood by those of ordinary skill in the art that the above description of the present invention is exemplary, and the exemplary embodiments disclosed herein can be easily modified into other specific forms without departing from the technical spirit or essential features of the present invention. Therefore, the exemplary embodiments described above should be interpreted as illustrative and not limited in any aspect.

### [Industrial Applicability]

Since a nanodispersion based on a pathogen cell wall skeleton of the present invention is easily dispersed in an aqueous solution, it can be prepared in various formulations of vaccines, and since it is mixed with various antigens and/or adjuvants, immunogenicity can be significantly increased, it can be used as immune activation therapeutic agents for various diseases. Therefore, it is expected to be widely applied to the field of vaccines used in the prevention or treatment of various diseases.

## Claims

1. Alive pathogen-mimetic nano-molecule, comprising:
a toll-like receptor 7 or 8 agonist, and a pathogen cell wall skeleton-based nanodispersion as active ingredients,
wherein the toll-like receptor 7 or 8 agonist is in an inactive state by binding of a lipid to an activation site.

2. The nano-molecule of claim 1, wherein the pathogen cell wall skeleton is a freeze-dried pathogen outer wall component dispersed in an aqueous solution.

3. The nano-molecule of claim 1, wherein the binding between the toll-like receptor 7 or 8 agonist and a lipid is cleavable.

4. The nano-molecule of claim 3, wherein the cleavable binding is any one or more selected from the group consisting of a carbamate bond, a disulfide bond, an ester bond, a peptide bond, an azide bond, an amide bond, a hydrazine bond, a thioether bond, a phosphodiester bond, a thioketal bond, and a combination thereof.

5. The nano-molecule of claim 1, wherein the toll-like receptor 7 or 8 agonist is any one or more selected from the group consisting of imidazoquinoline-, hydroxyadenine-, pteridine-, aminopyrimidine-, benzoazepine-, thia-oxoguanosine-based compounds, and a derivative thereof.

6. The nano-molecule of claim 1, wherein a chemical bond at the site of the binding between the toll-like receptor 7 or 8 agonist and a lipid is cleaved in response to a tumor microenvironment, or enzymes and pH of endosomes and lysosomes in cells, and the active site of the toll-like receptor 7 or 8 agonist is exposed so that a kinetic function is recovered within 4 days.

7. The nano-molecule of claim 1, wherein the nano-molecule has a diameter of 20 to 500 nm.

8. The nano-molecule of claim 1, wherein the nano-molecule is any one or more selected from the group consisting of a nanoliposome, a nanoemulsion, a nanomicelle, and a polymer nanoparticle.

9. The nano-molecule of claim 1, wherein the nano-molecule further comprises any one or more selected from the group consisting of a toll-like receptor agonist, a saponin, an antiviral peptide, an inflammasome inducer, a NOD ligand, a cytosolic DNA sensor (CDS) ligand, a stimulator of interferon genes (STING) ligand, an antigen, alum, a chemotherapeutic agent, an immune checkpoint inhibitor, and a combination thereof.

10. An adjuvant composition comprising the nano-molecule of claim 1 as an active ingredient.

11. A vaccine composition comprising the adjuvant composition of claim 10 and an antigen as active ingredients.

12. The vaccine composition of claim 11, wherein the antigen is one or more selected from the group consisting of a protein, a recombinant protein, a glycoprotein, a gene, a peptide, a polysaccharide, a lipopolysaccharide, a polynucleotide, cells, a cell lysate, bacteria, and viruses.

13. The vaccine composition of claim 11, wherein the vaccine composition further comprises a chemotherapeutic agent or an immune checkpoint inhibitor.

14. The vaccine composition of claim 11, wherein the vaccine composition is for preventing or treating cancer.

15. The vaccine composition of claim 14, wherein the vaccine composition inhibits cancer proliferation, metastasis or recurrence, or resistance to anticancer therapy.

16. A method of preparing a live pathogen-mimetic nano-molecule, comprising:
(a) disrupting pathogens;
(b) removing a lipid from the resulting lysate;
(c) removing a membrane protein from the lipid-removed lysate;
(d) isolating a pathogen cell wall skeleton by centrifuging the membrane protein-removed lysate;
(e) preparing a freeze-dried preparation by freeze-drying the isolated pathogen cell wall skeleton;
(f) mixing the freeze-dried preparation, a surfactant and a positively-charged buffer solution; and
(g) manufacturing a nano-molecule by adding a lipid, a lipid conjugate, or a toll-like receptor 7 or 8 agonist in which a lipid binds to an activation site to the mixture obtained in (f) and performing ultrasonication.

17. The method of claim 16, further comprising:
after (g), further adding any one or more selected from the group consisting of a toll-like receptor 7 or 8 agonist, a toll-like receptor agonist, a saponin, an antiviral peptide, an inflammasome inducer, a NOD ligand, a cytosolic DNA sensor (CDS) ligand, a stimulator of interferon genes (STING) ligand, an antigen, alum, a chemotherapeutic agent, an immune checkpoint inhibitor, and a combination thereof, and performing mixing and sonication.

18. The method of claim 16, wherein the surfactant of (f) is any one or more selected from the group consisting of sodium dodecyl sulfate (SDS), didodecyldimethylammonium bromide (DMAB), Pluronic F68, Pluronic F127, polyvinyl alcohol (PVA), Tween-80, Span-85, oleic acid, and a combination thereof.

19. The method of claim 16, wherein the positively-charged buffer solution of (f) is any one or more selected from the group consisting of an L-lysine (L-Lys) buffer solution, an L-arginine (L-Arg) buffer solution, an L-histidine (L-His) buffer solution, an L-tyrosine (L-Tyr) buffer solution, an L-aspartic acid (L-Asp) buffer solution, an L-glutamine (L-Glu) buffer solution, and a combination thereof.

20. A use of a composition comprising the nano-molecule of claim 1 as an active ingredient for preventing or treating a disease.

21. A method of preventing or treating a disease, comprising:
administering a composition comprising the nano-molecule of claim 1 as an active ingredient into a subject.
